# EUROPEAN PATENT APPLICATION

(11) **EP 1 982 726 A1**
(43) Date of publication of application: **22.10.2008**
(21) Application number: 07713896.4
(22) Date of filing: 07.02.2007
(51) Int. Cl.: A61K 39/00, A61K 9/127, A61K 39/002, A61K 39/005, A61K 39/02, A61K 39/085, A61K 39/112, A61K 39/35, A61K 47/24, A61P 31/04, A61P 33/00

(54) **NOVEL VACCINE CARRIER**

(30) Priority: 07.02.2006 JP 2006030246
(71) Applicant: Nippon Biologicals, Inc., Minato-Ku, Tokyo 107-0061 (JP)
(72) Inventor: WATARAI, Shinobu, Osaka 591-8037 (JP); KONO, Kenji, Osaka 590-0141 (JP)
(74) Representative: Denison, Christopher Marcus
(86) International application number: PCT/JP2007/052079
(87) International publication number: WO 2007/091580

(57) **Abstract**

An object of the present invention is to prepare novel vaccine carriers that can be used to produce vaccines that are capable of efficient induction of humoral and cellular immune responses. Another object of the present invention is to provide vaccines that are capable of efficient induction of humoral and cellular immune responses.

The present inventors revealed that the above-stated objects of the present invention could be attained by using liposomes containing succinylated poly(glycidol) and this finding has led to the accomplishment of the invention. Stated specifically, the present invention can attain the aforementioned objects by providing vaccine carriers comprising liposomes containing succinylated poly(glycidol).

SEQUENCE LISTING

<110> Nippon Biologicals, Inc.

<120> Novel Vaccination Carrier

<130> N-79-1 (YCT-1236)

<150> JP 2006-030246
<151> 2006-02-07

<160> 6

<170> PatentIn version 3.1

<210> 1
<211> 31
<212> DNA
<213> Artificial

<220>
<223> Forward primer for amplifying mouse IFN-gamma gene.

<400> 1
tgcatcttgg cttgcagctc ttcctcatgg c 31

<210> 2
<211> 32
<212> DNA
<213> Artificial

<220>
<223> Reverse primer for amplifying mouse IFN-gamma gene.

<400> 2
tggacctgtg ggttgttgac ctcaaacttg gc 32

<210> 3
<211> 32
<212> DNA
<213> Artificial

<220>
<223> Forward primer for amplifying mouse IL-4 gene.

<400> 3
ccagctagtt gtcatcctgc tcttctttct cg 32

<210> 4
<211> 32
<212> DNA
<213> Artificial

<220>
<223> Reverse primer for amplifying mouse IL-4 gene.

<400> 4
cagtgatgtg gacttggact cattcatggt gc 32

<210> 5
<211> 20
<212> DNA
<213> Artificial

<220>
<223> Forward primer for amplifying mouse G3PDH gene.

<400> 5
accacagtcc atgccatcac 20

<210> 6

<211> 20
<212> DNA
<213> Artificial

<220>
<223> Reverse primer for amplifying mouse G3PDH gene.

<400> 6
tccaccaccc tgttgctgta 20

## Description

### TECHNICAL FIELD

The present invention relates to novel vaccine carriers using liposomes having a fusogenic lipid membrane.

### BACKGROUND ART

The immune system of animals has the function of differentiating between self and non-self and eliminating the non-self from the body. The immunological reaction in a living body that is responsible for such discrimination between self and non-self is realized by cellular immunity that utilizes MHC class I molecules or by humoral immunity that utilizes MHC class II molecules. For instance, if a living body is infected with a virus or bacterium as an infectious pathogen, it tries to eliminate such infectious pathogens by using the above-mentioned cellular immunity or humoral immunity.

Vaccination is frequently utilized as a means for preventing those infectious pathogens. In the case of humans, as well as domesticated animals and companion animals, a great variety of vaccines are utilized.

Those vaccines are roughly divided into two types, inactivated vaccines (including toxoid vaccine) and attenuated vaccines. Inactivated vaccines are such vaccines that a pathogen or toxoid is treated with formalin or other chemicals to become noninfectious and then administered in an inactivated state or they are such vaccines that only the antigen portion of the pathogen is administered; examples of this type of vaccines that can be administered to humans include triple vaccines (DPT vaccine; diphtheria pertussis tetanus vaccine), Japanese encephalitis vaccine, influenza vaccine, tetanus toxoid vaccine, etc. Attenuated vaccines, on the other hand, are those vaccines which are administered in the form of pathogens that are weakly virulent but do have infectivity, as exemplified by naturally occurring attenuated strains or artificially created strains of attenuated variants; examples of this type of vaccines that can be administered to humans include BCG vaccine, poliomyelitis vaccine, measles vaccine, rubella vaccine, mumps vaccine, varicella vaccine (chickenpox vaccine), etc.

Inactivated vaccines have the advantage of being less likely to cause pathogen-mediated infection and side effects as the result of their administration but, on the other hand, they are characterized by the ability to acquire only humoral immunity. Attenuated vaccines, on the other hand, use live pathogens, so they have the disadvantage that their virulence might for some reason be restored to develop side effects. What is more, except in the case of some attenuated vaccines (such as poliomyelitis vaccine) that are to be administered orally, vaccines are generally administered by intramuscular injection, so they have additional problems in that the antibody titers of IgG antibodies may increase but those of antibodies in other classes (such as IgA and IgM) will not and that they cannot fully induce the cellular immune response which is important for protection against infection.

Infection with infectious pathogens starts with those pathogens invading the body from mucosal surfaces as in the nasal, tracheal, intestinal and ocular mucosa, so if cellular immunity can be induced by vaccination, the invasion of pathogens into the body can be halted at the border. Although the mucosal membranes in the living body cover the surfaces of tract lumens such as oral cavity, nasal cavity, digestive tract and reproductive organs, as well as the mucosal surfaces of the eyes, what is constantly functioning on those surfaces is mucosal immunity that mainly involves secretory IgA and mucosa-associated lymph tissues against pathogenic microorganisms (e.g. viruses and bacteria), dietary antigens, and non-self foreign substance to which the mucosal membranes are constantly exposed. The mucosal immunity halts the invasion of those non-self foreign substances into the body by exhibiting diverse actions such as suppression of incorporation of protein antigens from the mucosal surfaces, inhibition of the adsorption of bacteria or viruses on the mucosal epithelia, and neutralization of viruses with which epithelial cells have been infected.

However, as mentioned above, many cases of the conventional vaccination have had the problem of failing to increase the antibody titers of antibodies in non-IgG classes (such as IgA and IgM) or to fully induce the cellular immune response, so no immune response that involves the production of antigen-specific antibodies (secretory IgA) can be effectively induced on mucosal surfaces which are sites of infection with infectious pathogens. To solve these problems, studies have been made on the assumption that antigen-specific immune response could be induced both systemically and on the mucosal surfaces at various parts of the body by administering antigens via mucosal surfaces as in oral immunization or nasal immunization.

With a view to imparting such mucosal immunity, attempts have been made to incorporate antigens in liposomes and administering them to mucosal membranes as vaccines. It was shown, for example, that by incorporating a *Salmonella enterica* serovar Enteritidis antigen as an immunogen in liposomes and dropping it onto the eyes of chickens, systemic immune response could be induced to thereby inhibit the invasion of *Salmonella enterica* serovar Enteritidis through the intestinal lumen (Non-Patent Document 1 and Non-Patent Document 2).

However, in the field of vaccine production, it is desired to develop vaccine carriers that can achieve even more efficient increases in the antibody titers of various classes of antibodies (in humoral immune response) and in cellular immune response.
Non-Patent Document 1: Fukutome, K. et al., Development and Comparative Immunology, 25, 2001, 475-484;.
Non-Patent Document 2: Li, W. et al, Development and Comparative Immunology, 28, 2004, 29-38.

### DISCLOSURE OF THE INVENTION

### PROBLEMS TO BE SOLVED BY THE INVENTION

An object of the present invention is to prepare novel vaccine carriers that can be used to produce vaccines that are capable of efficient induction of humoral and cellular immune responses. Another object of the present invention is to provide vaccines that are capable of efficient induction of humoral and cellular immune responses.

### MEANS FOR SOLVING THE PROBLEMS

The present inventors revealed that the above-stated problems of the present invention could be solved by using liposomes containing a fusogenic lipid (succinylated poly(glycidol)) and this finding has led to the accomplishment of the invention. Stated specifically, the present invention can solve the aforementioned problems by providing vaccine carriers comprising liposomes containing succinylated poly(glycidol).

### EFFECTS OF THE INVENTION

By using the above-described vaccine carriers that comprise liposomes containing succinylated poly(glycidol), efficient vaccines can be obtained that achieve significant increases in antibody titers as compared with the case of using vaccine carriers that comprise the conventional liposomes. In addition, the vaccines prepared by using the above-described vaccine carriers are capable of efficient induction of not only humoral immunity but also cellular immunity.

### BRIEF DESCRIPTION OF THE DRAWINGS

[FIG. 1]

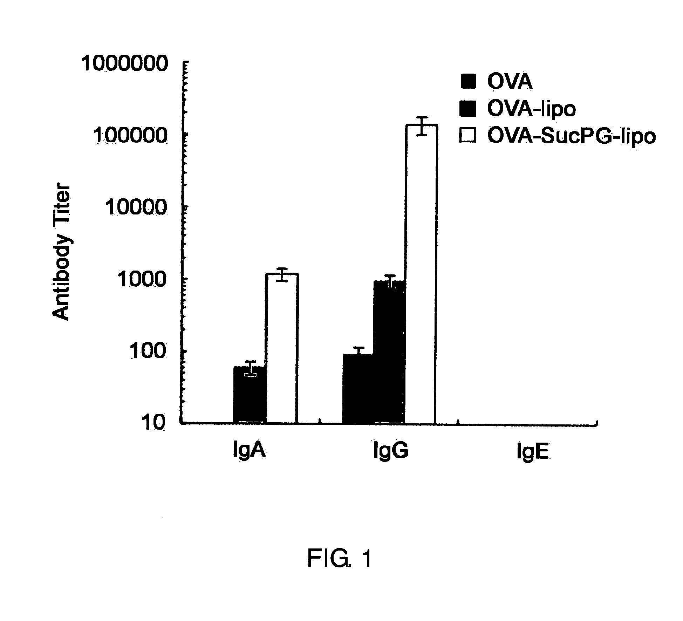

   FIG. 1 is a graph showing the antibody titers of OVA-specific IgM antibody, IgG antibody and IgE antibody in serum for the case where BALB/c mice were intraperitoneally immunized with a vaccine made up of OVA-SucPG-liposomes, a vaccine made up of OVA-liposomes, and a vaccine solely composed of OVA.
[FIG. 2]

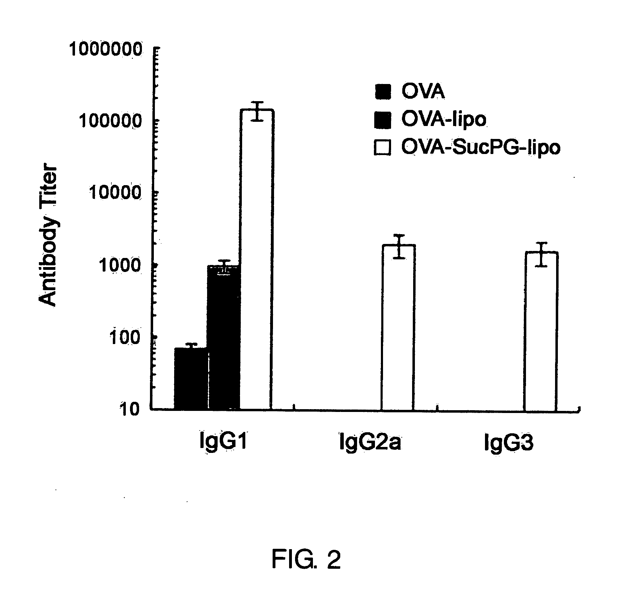

   FIG. 2 is a graph showing the antibody titers of subclasses of OVA-specific IgG antibody (IgG1 IgG2a, and IgG3) in serum for the case where BALB/c mice were intraperitoneally immunized with a vaccine made up of OVA-SucPG-liposomes, a vaccine made up of OVA-liposomes, and a vaccine solely composed of OVA.
[FIG. 3]

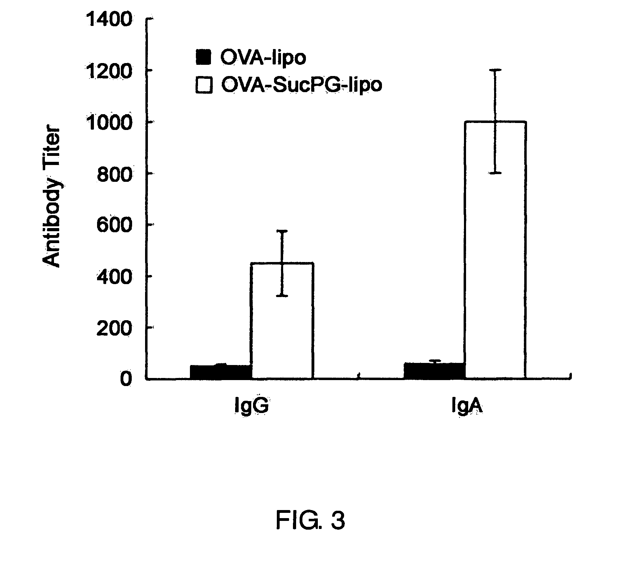

   FIG. 3 is a graph showing the results of antibody class induction in the intestinal fluid for the case of transnasal immunization.
[FIG. 4]

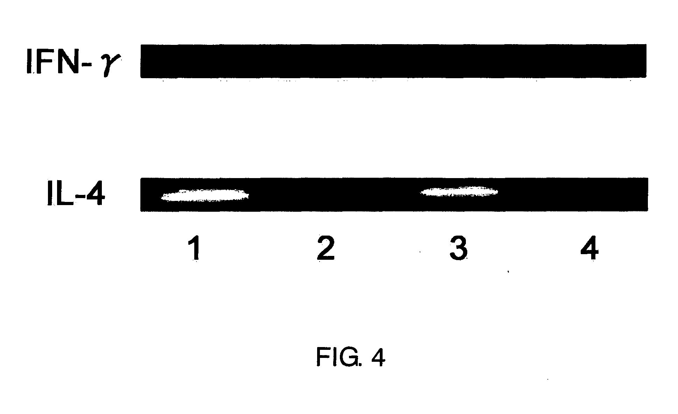

   FIG. 4 shows the results of examination of mRNA expression of IFN-γ gene and IL-4 gene in spleen lymphocytes from mice immunized intraperitoneally with a vaccine made up of OVA-SucPG-liposomes using the RT-PCR technique.
[FIG. 5]

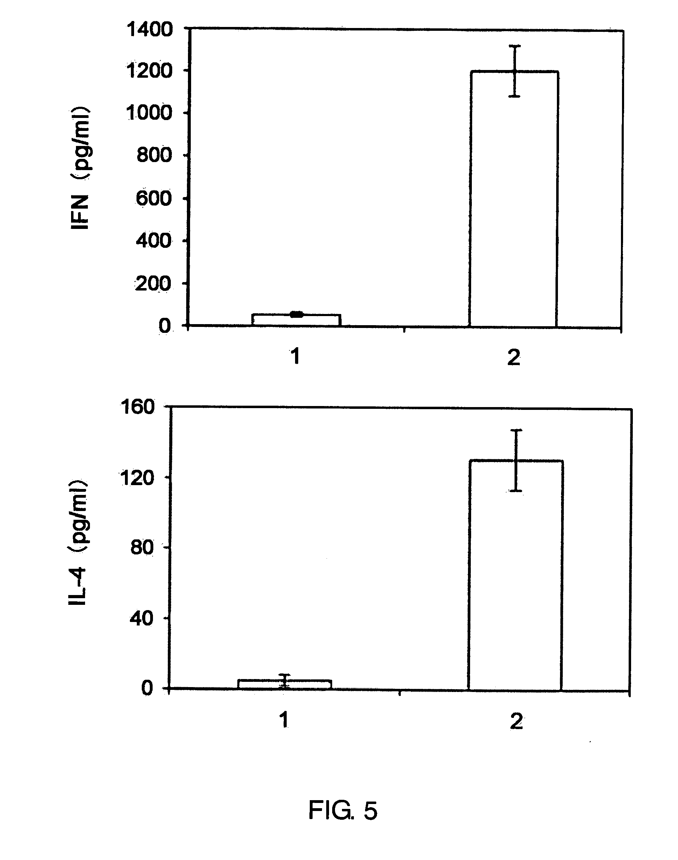

   FIG. 5 shows in graphs the results of quantitation of IFN-γ and IL-4 in the culture supernatant of spleen lymphocytes from mice immunized intraperitoneally with a vaccine made up of OVA-SucPG-liposomes.
[FIG. 6]

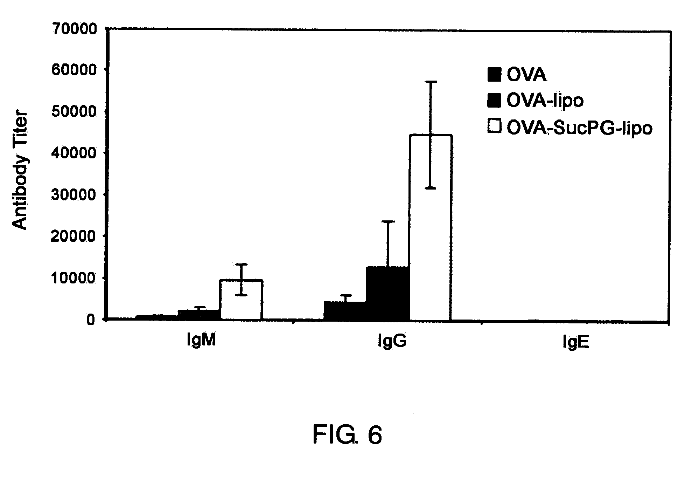

   FIG. 6 is a graph showing the antibody titers of OVA-specific IgM antibody, IgG antibody and IgE antibody in serum for the case where BALB/c mice were intraperitoneally immunized with a vaccine made up of OVA-SucPG-liposomes, a vaccine made up of OVA-liposomes, and a vaccine solely composed of OVA.
[FIG. 7]

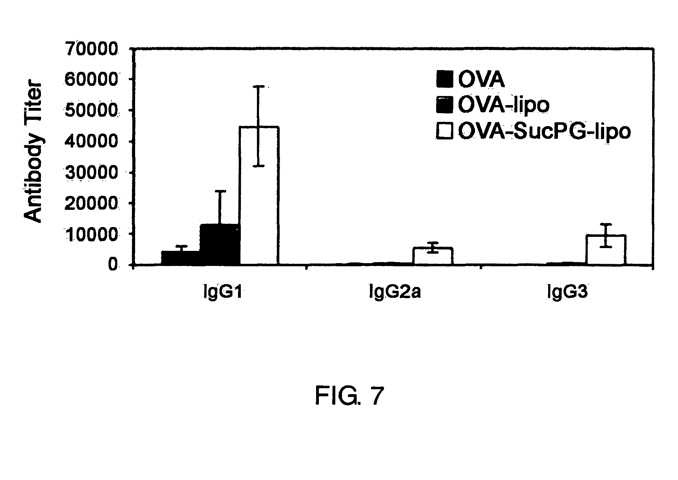

   FIG. 7 is a graph showing the antibody titers of subclasses of OVA-specific IgG antibody (IgG1, IgG2a, and IgG3) in serum for the case where BALB/c mice were intraperitoneally immunized with a vaccine made up of OVA-SucPG-liposomes, a vaccine made up of OVA-liposomes, and a vaccine solely composed of OVA.
[FIG. 8]

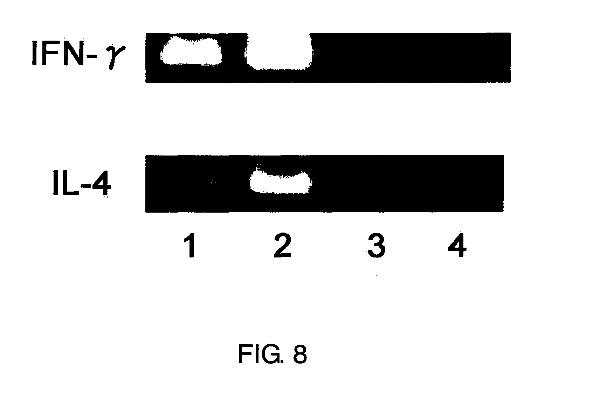

   FIG. 8 shows the results of examination of mRNA expression of IFN-γ gene and IL-4 gene in spleen lymphocytes from mice immunized intraperitoneally with a vaccine made up of OVA-SucPG-liposomes using the RT-PCR technique.
[FIG. 9]

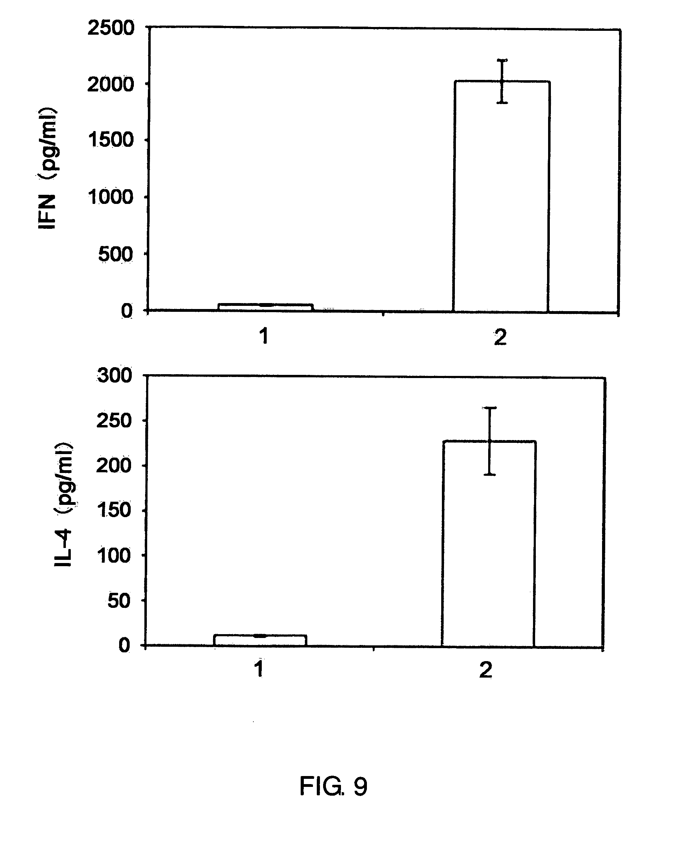

   FIG. 9 shows in graphs the results of quantitation of IFN-γ gene and IL-4 gene in the culture supernatant of spleen lymphocytes from mice immunized intraperitoneally with a vaccine made up of OVA-SucPG-liposomes.
[FIG. 10]

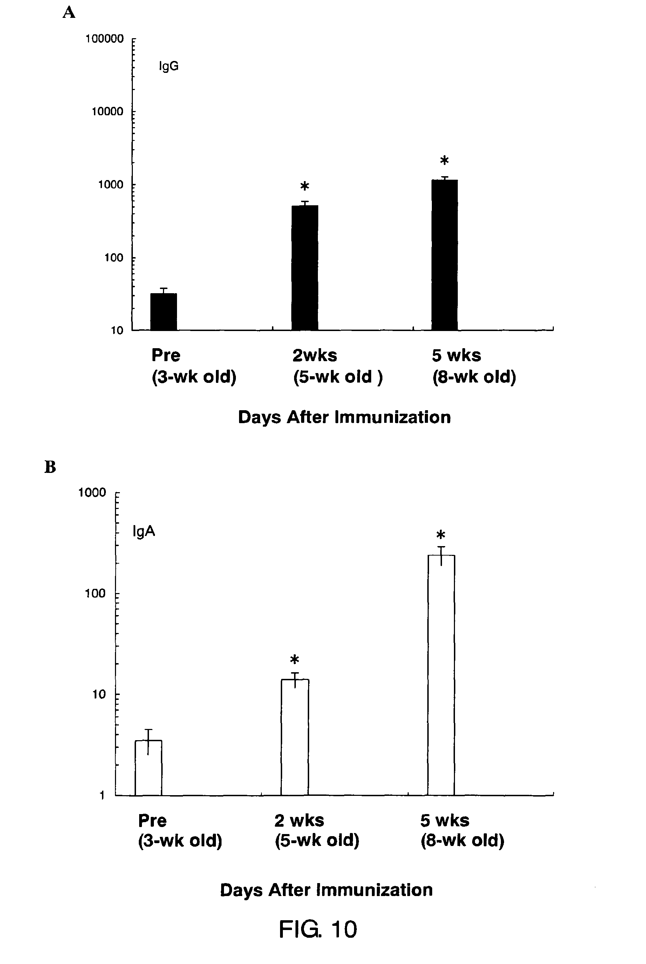

   FIG. 10 shows in graphs the results of antibody titer antibodies in the blood from chickens immunized by ophthalmic administration of a vaccine made up of *Salmonella enteritidis* antigen-SucPG-liposomes.
[FIG. 11]

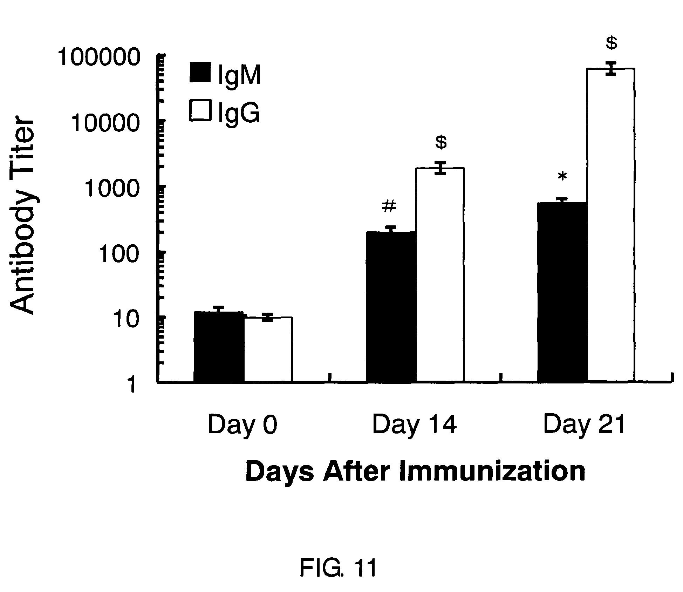

   FIG. 11 is a graph showing the results of antibody titer antibodies in the blood from mice immunized by transnasal administration of a vaccine made up of *Trypanosoma brucei* antigen-SucPG-liposomes.
[FIG. 12]

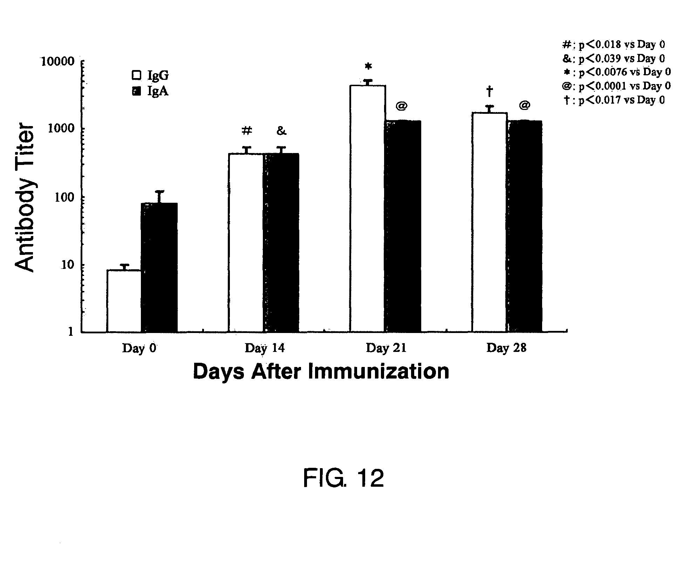

   FIG. 12 is a graph showing the results of antibody titer antibodies in the blood from cows immunized by transnasal administration of a vaccine made up of *Staphylococcus aureus* antigen-SucPG-liposomes.
[FIG. 13]

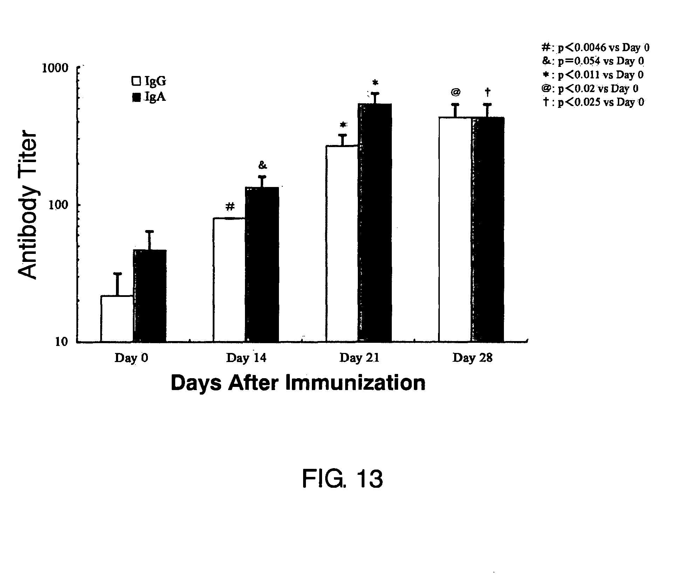

   FIG. 13 is a graph showing the results of antibody titer antibodies in the milk from cows immunized by transnasal administration of a vaccine made up of *Staphylococcus aureus* antigen-SucPG-liposomes.
[FIG. 14]

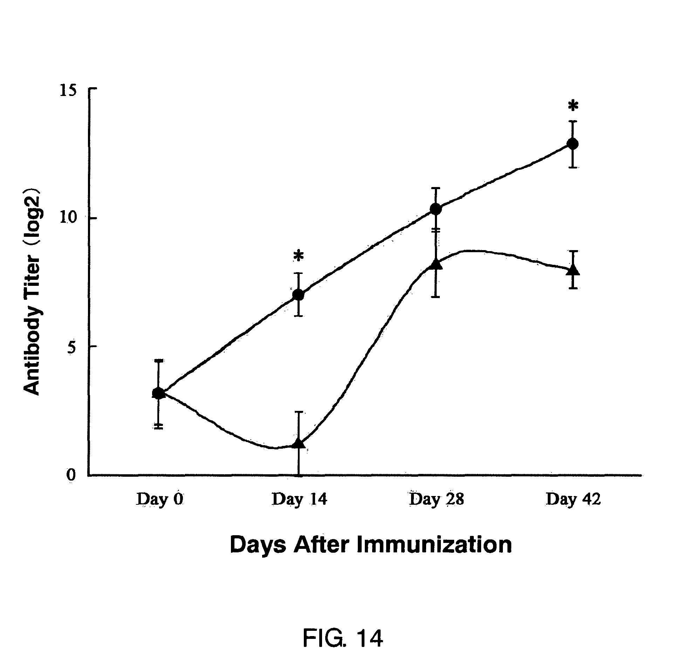

   FIG. 14 is a graph showing the results of antibody titer antibodies in the blood from carp immunized by oral administration of a vaccine made up of *Aeromonas salmonicida* antigen-SucPG-liposomes.
[FIG. 15]

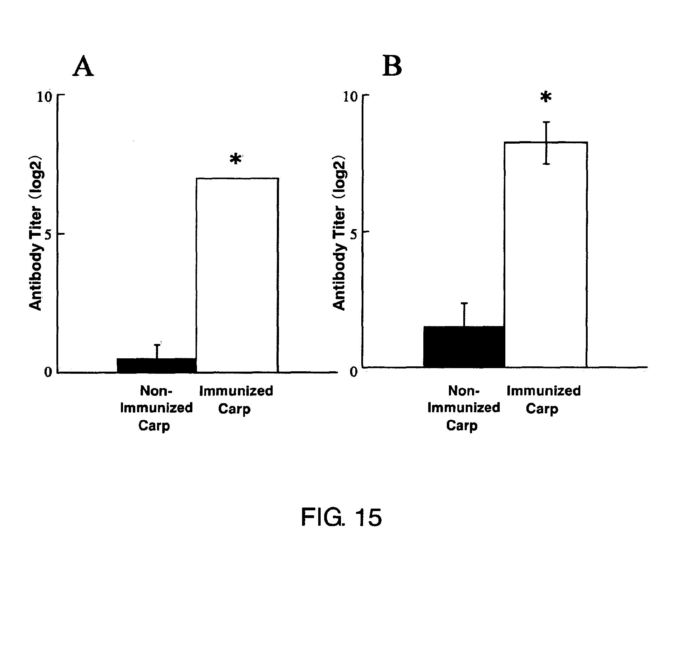

   FIG. 15 shows in graphs the results of antibody titer antibodies in the intestinal fluid and bile from carp immunized by oral administration of a vaccine made up of *Aeromonas salmonicida* antigen-SucPG-liposomes.
[FIG. 16]

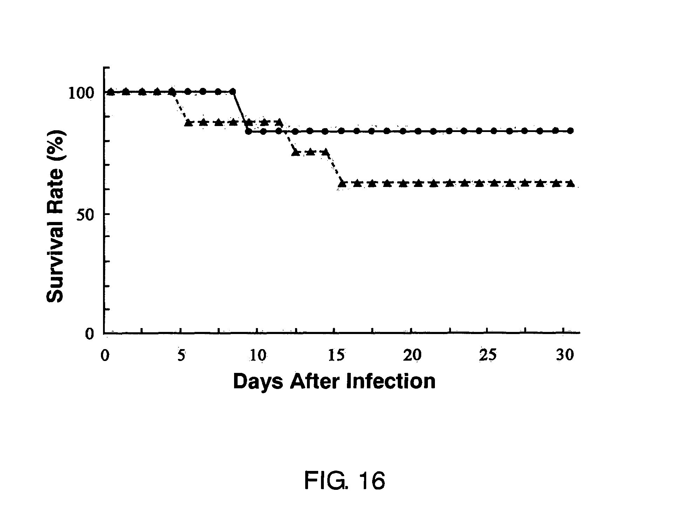

   FIG. 16 is a graph showing the transitional change in survival rate of carp immunized by oral administration of a vaccine made up of *Aeromonas salmonicida* antigen-SucPG-liposomes.
[FIG. 17]

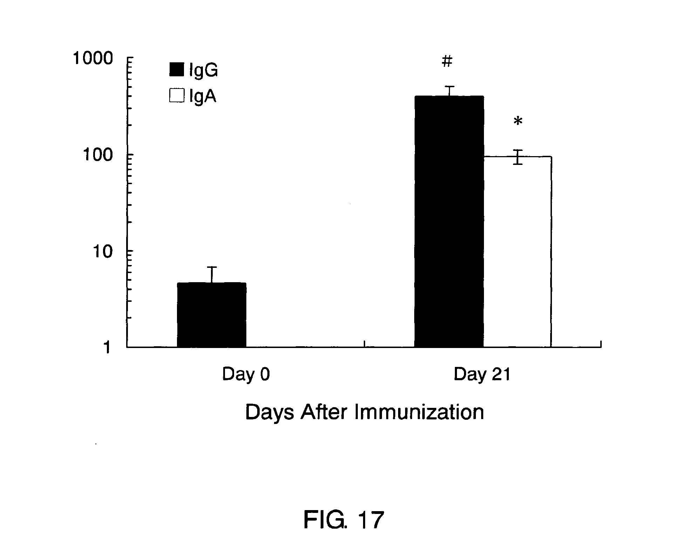

   FIG. 17 is a graph showing the results of antibody titer antibodies in the blood from mice immunized by transnasal administration of a vaccine made up of *Mycoplasma gallisepticum* antigen-SucPG-liposomes.
[FIG. 18]

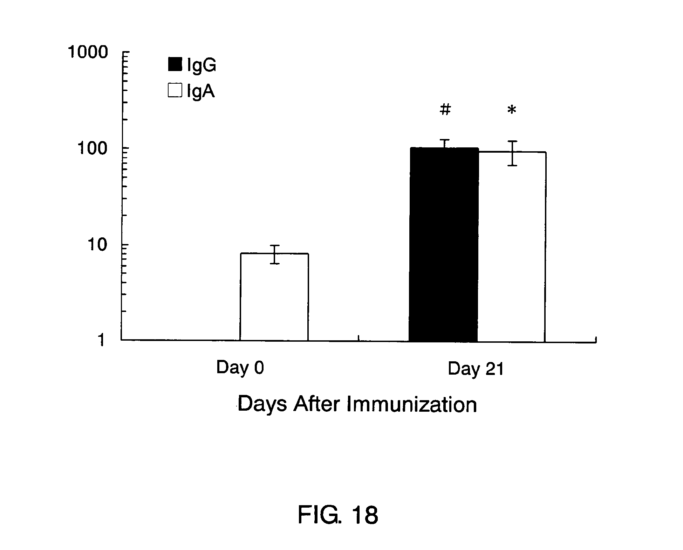

   FIG. 18 is a graph showing the results of antibody titer antibodies in the blood from mice immunized by transnasal administration of a vaccine made up of Newcastle disease virus antigen-SucPG-liposomes.

### MODES FOR CARRYING OUT THE INVENTION

As described above, the present invention is characterized by providing vaccine carriers comprising liposomes that contain succinylated poly(glycidol) (SucPG). The advantage of using the vaccine carriers comprising such liposomes is that irrespective of whether the immunogen contained in the liposomes is an inactivated vaccine or an attenuated vaccine and whatever is the route of administration, not only the antibody titers of IgG antibodies but those of other classes of antibodies can also be elevated and what is more, cellular immunity as well as humoral immunity can also be induced.

The cellular immunity that is induced from the use of the vaccine carrier of the present invention which comprises liposomes containing succinylated poly(glycidol) (SucPG) is believed to have been accomplished by internalizing the immunogen within antigen-presenting cells. To be more specific, using the vaccine carrier of the present invention, one can incorporate the immunogen into the lumens of liposomes and can hence internalize the immunogen within the antigen-presenting cells. As a result, so it was assumed, the immunogen incorporated into the antigen-presenting cell combined as a self-component with an MHC class I molecule and presented itself as an antigen on the antigen-presenting cell, eventually inducing cellular immunity.

The succinylated poly(glycidol) (SucPG) as used herein is an amphiphilic compound characterized by having an alkyl group. Having an alkyl group, SucPG can be anchored to a liposome membrane. The alkyl group in SucPG preferably contains 6 to 24 carbon atoms and, more preferably, contains alkyl groups having 6 to 18 carbon atoms. The most preferred alkyl group is an n-decyl group having 10 carbon atoms. SucPG has the skeleton of the main chain being similar to those of amphiphilic polyethylene glycol and side chains with a carboxyl group, so it is characterized in that it stabilizes the liposome membrane in a neutral environment but that in an acidic environment, the carboxyl group on side chains is protonated to induce membrane fusion. By incorporating the SucPG into the liposome membrane, the resulting liposome (SucPG-liposome) comes to develop fusogenicity in an acidic environment. In other words, when the SucPG-liposome is incorporated into the antigen-presenting cell by endocytosis, the pH in the lysosome drops. Then the SucPG-liposome exhibits its fusogenicity and fuses with the lysosome membrane to cause the encapsulated antigenic substance to be released into the cytoplasm (internalization of the antigen).

The SucPG that is to be used in the present invention can be prepared by reacting the synthetic polymer poly(glycidol) with succinic anhydride in N,N-dimethylformamide at 80°C for 6 hours.

What is characteristic of the present invention is that succinylated poly(glycidol) is added to the lipid that composes the liposome used in the vaccine carrier. To be more specific, the vaccine carrier of the present invention contains succinylated poly(glycidol) in an amount of 10 to 40 wt%, preferably 20 to 35 wt%, most preferably 30 wt%, of the lipid that composes the liposome.

The vaccine carrier of the present invention can be used for transmucosal administration of the immunogen contained within the liposome. The term "mucosa" or "mucosal membrane" as used herein collectively refers to sites that cover the inner surfaces of the lumens of hollow viscera such as the digestive organs, respiratory organs, and genitourinary organs and their free surfaces are always wet with secretions from mucosal glands and goblet cells. Mucosal membranes to which the vaccine carrier of the present invention can be applied include membranes of the oral cavity, throat, nasal cavity, aural cavity, conjunctival sac, vagina, and anus. By applying the immunogen-containing liposome to these mucosal surfaces, the immunogen can be incorporated into the body via the mucosal surfaces.

The vaccine carrier of the present invention can also be used for delivery by non-transmucosal administration of the immunogen contained within the liposome of the vaccine carrier. In the present invention, routes other than the transmucosal route may include intraperitoneal administration of the immunogen contained within the liposome of the vaccine carrier. For instance, when the immunogen is administered intraperitoneally, it can be incorporated into the body from the surfaces of organs in the abdominal cavity, as exemplified by the gastrointestinal tract, genital organs, liver, and pancreas. By thus administering the immunogen into the body, the immunogen can be incorporated into antigen-presenting cells ubiquitously present in the body.

Lipids that compose the liposome in the present invention include, for example, phosphatidylcholines, phosphatidylethanolamines, phosphatidylserines, phosphatidic acids or long-chain alkyl phosphates or phosphatidylglycerols, and cholesterols (Chol). When preparing liposomes in the present invention, the lipids listed above may be used either independently or in combination of any two or more of those lipids.

Phophatidylcholines that are used as lipids for composing the liposome in the present invention include dimyristoyl phosphatidylcholine (DMPC), dipalmitoyl phosphatidylcholine (DPPC), distearoyl phosphatidylcholine (DSPC), dioleyl phosphatidylcholine (DOPC), yolk lecithin (egg PC), etc.

Phophatidylethanolamines that are used as lipids for composing the liposome in the present invention include dioleyl phosphatidylethanolamine (DOPE), dimyristoyl phosphatidylethanolamine, dipalmityol phosphatidylethanolamine, distearoyl phosphatidylethanolamine (DSPE), etc.

Phophatidylserines that are used as lipids for composing the liposome in the present invention include dioleyl phosphatidylserine (DOPS), dipalmitoyl phosphatidylserine (DPPS), etc.

Phophatidic acids or long-chain alkyl phosphates that are used as lipids for composing the liposome in the present invention include dimyristoyl phosphatidic acid, dipalmitoyl phosphatidic acid, distearoyl phosphatidic acid, dicetyl phosophate, etc.

Phophatidylglycerols that are used as lipids for composing the liposome in the present invention include dimyristoyl phosphatidylglycerol, dipalmitoyl phosphatidylglycerol, distearoyl phosphatidylglycerol, etc.

Among the compounds listed above, those which are particularly preferred for use are DOPE, DPPC, DSPC, DPPS, DSPE, Chol, etc.

When the above-listed lipids are to be used in admixture, the proportions at which the respective lipids are incorporated can be determined as appropriate by the desired size of liposomes, the desired fluidity, etc. In the present invention, liposomes are preferably prepared by mixing DOPE and DPPC at 1:1.

Liposomes are classified as MLV (multilamellar vesicles), DRV (dehydration-rehydration vesicles), LUV (large unilamellar vesicles) or SUV (small unilamellar vesicles), etc. depending on their structures or the method of their preparation. The liposome of the present invention which contains succinylated poly(glycidol) (SucPG) is also available in various types of liposomes including MLV, DRV, LUV and SUV that are composed of multiple layers.

To prepare the SucPG-containing liposomes, any conventionally known methods of liposome production may be employed. A variety of methods for liposome production have heretofore been known in the technical field of interest.

To give examples of some common methods for liposome production, the following may be mentioned: (1) a lipid is dissolved in a suitable organic solvent (such as, chloroform, ether, etc.) and the solvent is distilled off under vacuum to form a thin lipid film, which is then hydrated (or swollen) in water by mechanical agitating means; (2) a lipid is dissolved in an organic solvent (such as ether or ethanol) and the resulting solution is injected into high-temperature warmed water by suitable means (such as a syringe or a nozzle) under pressure at a constant rate, in the process of which the organic solvent is distilled off or diluted, whereby the lipid forms a double layer to prepare liposomes; (3) a lipid is mixed with a surfactant (such as cholic acid or deoxycholic acid) to form micelles in an aqueous solution and the resulting micelle solution is deprived of the surfactant (such as cholic acid or deoxycholic acid) by a suitable operation such as dialysis or gel filtration so as to prepare liposomes; (4) an organic solvent having a lipid dissolved therein is added to an aqueous phase, which is sonicated to form a W/O emulsion which is then deprived of the organic solvent to form a gel which is allowed to undergo phase inversion by mechanical agitation so as to prepare liposomes; (5) a thin film of lipid is mixed with an aqueous solvent so that it is hydrated or swollen and the container is mechanically vibrated to separate the thin lipid film from its inner surfaces and, thereafter, the separated thin lipid film is sonicated or passed through an orifice of a given size by means of a French press, a pressurized filtering apparatus, or an extruder so as to prepare liposomes; and (6) liposomes are freeze-dried and then rehydrated with an aqueous solvent to thereby prepare liposomes.

With a view to supplementing the immuno-augmenting activity, the vaccine carrier of the present invention may further contain an adjuvant. Adjuvants that can be contained in the vaccine carrier of the present invention include monophosphoryl lipid A, cytokine, lectin, etc.

The immunogen that can be contained in the vaccine carrier of the present invention may include (but not limited to) any of the immunogens with which humans or animals (mammals, fishes, etc.) are desirably vaccinated. Examples of the immunogens include immunogens derived from bacteria, immunogens derived from viruses, and immunogens derived from protozoa.

In the case, for example, that the case of applying the vaccine carrier of the present invention is applied to immunogens in humans, the following may be contained in the vaccine carrier: a virus-derived immunogen selected from among an influenza virus antigen, a SARS virus antigen, an AIDS virus antigen and the like; or a bacterium-derived immunogen selected from among pathogenic *Escherichia coli* O-157 antigen, *Salmonella* antigen, *Staphylococcus aureus* antigen, *Aeromonas* antigen, tubercule bacillus antigen and the like; or a protozoan-derived immunogen selected from among trypasonoma antigen, coccidium antigen, malaria antigen, theileria antigen and the like.

Consider then the case of applying the vaccine carrier of the present invention to immunogens in animals; in this case, any one of the antigens derived from pathogens of important infectious diseases in domestic animals may be contained and examples of the antigens include:
for chickens, bacterium-derived immunogens such as *Salmonella enterica* serovar Enteritidis antigen, *Haemophilus paragallinarum* antigen; virus-derived immunogens such as chick influenza virus antigen, Newcastle disease virus antigen, infectious bronchitis virus antigen; protozoan-derived immunogens such as leucocytozoon antigen, eimeria antigen;
for pigs, a virus-derived immunogen such as infectious gastroenteritis virus antigen; a bacterium-derived immunogen such as *Bordetella bronchiseptica* antigen; and protozoan-derived immunogens such as toxoplasma antigen, eimeria antigen;
for cows, a virus-derived immunogen such as bovine viral diarrhea/mucosal disease virus antigen; bacterium-derived immunogens such as *Staphylococcus aureus* antigen and *Mycobacterium avium* var paratuberculosis antigen; and protozoan-derived immunogens such as theileria antigen, babesia antigen and the like;
for horses, virus-derived immunogens such as equine rhinopneumonitis virus antigen and equine influenza virus antigen; and protozoan-derived immunogens such as trypanosoma antigen, babesia antigen; and
for fishes, bacterium-derived immunogens such as vibrio antigen, aeromonus antigen; virus-derived immunogens such as infectious pancreatic necrosis virus antigen, iridovirus antigen; and protozoan-derived immunogens such as ichthyobodo protozoan antigen, hexamita protozoan antigen.

### EXAMPLES

### Example 1: Preparation of Succinylated Poly(glycidol) (SucPG) Liposomes

To a lipid composition consisting of dipalmitoyl phosphatidylcholine (DPPC) (Sigma) and dioleyl phosphatidylethanolamine (DOPE) (Sigma) at a molar ratio of 1:1 (each being 10 µmoles), SucPG was added at a lipid weight ratio of 10%, 20% or 30% to prepare three kinds of SucPG-containing liposomes with different SucPG concentrations. The SucPG to be used in Example 1 was of a type having a C₁₀ n-decyl group as an alkyl group and it was synthesized by a documented method (Kono K. et al., J. Controlled Release, 68, 225-235 (2000); Kono K. et al., Biochim. Biophys. Acta, 1325, 143-154 (1997); or Kono K. et al., Biochim. Biophys. Acta, 1193, 1-9 (1994)). Specifically, poly(epichlorohydrin) was subjected to reaction in dimethylformamide in the presence of potassium acetate at 175°C for 6 hours to prepare poly-glycidyl acetate, which in turn was subjected to reaction in methyl carbitol in the presence of potassium acetate at 150°C for 1 hour to synthesize poly(glycidol). The thus synthesized polymer poly(glycidol) was reacted with succinic anhydride in N,N-dimethylformamide at 80°C for 6 hours to prepare SucPG.

To prepare liposomes, the procedure described in Non-Patent Document 1 may be adopted. Specifically, 2 µmoles of DPPC, 2 µmoles of DOPE, and SucPG were dissolved in an organic solvent and mixed in a conical flask. The lipids were dried on a rotary evaporator and placed for 30 minutes under vacuum in a desiccator. As a model antigen, 4 mg/mL of ovalbumin (OVA) was added and the mixture was incubated at 35-40°C for 3 minutes, followed by vigorous vortexing to disperse the lipid film. In this way, the model antigen OVA was encapsulated in the liposomes. Any OVA that was not encapsulated in the liposomes was removed by repeated centrifuging at 14000 g for 20 minutes at 4°C so as to purify the liposomes having the model antigen OVA encapsulated therein. Prepared in this way were multilamellar vesicles (OVA-SucPG-liposomes) (MLV).

### Example 2: Study of Immune Response from Transmucosal Administration of Vaccine in Succinylated Poly(glycidol) (SucPG) Liposomes

The purpose of this Example was to study the immune response from transmucosal administration of a vaccine in the SucPG-containing liposomes prepared in Example 1, as compared with a vaccine in the SucPG-free liposomes.

The vaccine in the SucPG-containing liposomes was prepared as described in Example 1. On the other hand, the vaccine in the SucPG-free liposomes was a vaccine in multilamellar vesicles (OVA-liposomes) (MLV) that were prepared by encapsulating OVA in a lipid composition consisting of DPPC and DOPE at a molar ratio of 1:1.

The thus prepared two types of vaccines, one in the OVA-SucPG-liposomes and the other in the OVA-liposomes, as well as a vaccine solely composed of OVA were administered transnasally to BALB/c mice twice at a 7-day interval, each time to give 100 µg per mouse of OVA.

Seven days after the final administration of the immunogen, 0.1 ml of blood was taken from the orbital venous plexus and the serum collected from the blood was used to study the production of anti-OVA antibodies (IgM, IgG, and IgE) by the ELISA procedure. Also, seven days after the final administration, the intestinal fluid was collected and studied for the production of anti-OVA antibodies (IgA and IgG) by the ELISA procedure. In addition, the immune serum obtained was used to make an analysis for anti-OVA-IgG subclasses by the ELISA procedure.

The results are shown in FIGs. 1 to 3. FIG. 1 shows the results of antibody class induction in the serum as regards the immune response from transnasal immunization of the BALB/c mice with the vaccine in the OVA-SucPG-liposomes, the vaccine in the OVA-liposomes, and the vaccine solely composed of OVA; the black column shows the result of immunization with OVA only (OVA); the gray columns show the result of immunization with the OVA-liposomes (OVA-lipo); and the white columns show the result of immunization with the OVA-SucPG-liposomes (OVA-SucPG-lipo). FIG. 2 shows the specific results with the anti-OVA-IgG antibody subclasses that were induced in the serum. FIG. 3 shows the results of antibody class induction in the intestinal fluid for the case of transnasal immunization. In FIGs. 2 and 3, the immunogens associated with the respective columns are the same as explained above in connection with FIG. 1.

From the results shown in FIG. 1, it can be seen that when the BALB/c mice were immunized intranasally with the variety of immunogens, antibodies of IgA and IgG classes were produced in the animal bodies, with the production of the IgG class antibody being generally greater than that of the IgA class antibody. For each class of antibody, the relationship between the type of vaccine carrier and the amount of antibody production was investigated; it was shown that, in comparison with the case of immunization with the vaccine solely composed of OVA or with the vaccine in the OVA-liposomes, immunization with the vaccine in the OVA-SucPG-liposomes was capable of antibody production in a significantly high efficiency (p<0.0027).

In addition, the results shown in FIG. 2 indicate that immunization with the vaccine in the OVA-SucPG-liposomes could induce not only IgG1 which was an IgG subclass of Th2 (humoral immunity) type but also IgG2a and IgG3 which were IgG subclasses of Th1 (cellular immunity) type. On the other hand, when immunization was effected using the vaccine in the OVA-liposomes or the vaccine solely composed of OVA, IgG1 was practically all the antibody that cold be produced. Further, as for the amount of production of that IgG1 antibody, it was shown that immunization with the vaccine in the OVA-SucPG-liposomes was capable of antibody production in a significantly high efficiency (p<0.011) as compared with the case of immunization with the vaccines in other vaccine carriers (the vaccine in the OVA-liposomes and the vaccine solely composed of OVA).

Further in addition, FIG. 3 shows that transnasal use of the vaccine in the OVA-SucPG-liposomes was capable of efficient antibody production in the intestinal fluid as compared with the vaccine in the OVA-liposomes.

Accordingly, the following general observations were obtained from the transmucosal administration of the vaccine in the SucPG-containing liposomes: it was capable of efficient antigen introduction into antigen-presenting cells, eventually inducing high antibody production in the blood as well as high antibody production in the intestinal tract; and it was potentially capable of inducing not only humoral immunity but also cellular immune response.

### Example 3: Induction of Cellular Immune Response from Transmucosal Administration of Vaccine in SucPG-Containing Liposomes

Since it was shown in Example 2 that immunization with the vaccine in the SucPG-containing liposomes by transmucosal administration of the antigen had the potential to induce cellular immune response, Example 3 was conducted to study an ability to exert the cellular immune response for the case of using the vaccine in the SucPG-containing liposomes.

The vaccine in the OVA-SucPG-liposomes prepared in Example 1 was administered transnasally to BALB/c mice twice at a 7-day interval, each time to give 100 µg per mouse of OVA. Seven days after the final administration, the mice were sacrificed and the spleen was collected and subjected to density-gradient centrifugation to purify the spleen lymphocytes.

From the purified spleen lymphocytes, total RNA was extracted using TRIzol^{™} (Invitrogen) and IFN-γ which was an index of cellular immunity and IL-4 were checked for mRNA expression by the RT-PCR technique.

First of all, cDNA was synthesized from the total RNA. The total RNA (1 µg to 5 µg) was mixed with an oligo(dT)₁₂₋₁₈ primer (500 ng) and dNTP Mix (10 nmol) to make a total volume of 12 µL; the mixture was subjected to reaction at 65°C for 5 minutes and quenched on ice. Subsequently, 4 µL of 5 x First-Strand Buffer, 2 µL of 0.1 M DTT and 1 µL of RNaseOUT^{™} Recombinant Ribonuclease Inhibitor (40 units/µL) (Invitrogen) were added and, following a 2-minute reaction at 42°C, SuperScript^{™} II reverse transcriptase (Invitrogen) was added in an amount of 200 units. Following an additional 50-minute reaction at 42°C, the reverse transcriptase was inactivated by performing a reaction at 70°C for 15 minutes.

One microliter of the resulting cDNA, 2.5 pmol of each of the primers identified below, 37.5 nmol of MgCl₂, 10 nmol of dNTP Mix, and 1 unit of Taq DNA Polymerase (Invitrogen) were added to a PCR buffer to make a total volume of 25 µL and PCR reaction was performed in TaKaRa PCR Thermal Cycler MP TP3000 (TaKaRa). The PCR reaction consisted of a 5-minute reaction at 94°C, followed by 35 cycles, in which each consisting of a 94°C x 45-sec reaction, a 60°C x 45-sec reaction and a 72°C x 2-min reaction, and the final reaction at 72°C for 7 minutes. Following the PCR, the sample was electrophoresed on a 2% agarose gel and made visible by staining with ethidium bromide.

In Example 3, the following primers were used to amplify the mouse IFN-γ gene:
forward primer: 5'-tgcatcttggcttgcagctcttcctcatggc-3' (SEQ ID NO: 1) and
reverse primer: 5'-tggacctgtgggttgttgacctcaaacttggc-3' (SEQ ID NO: 2) ;
and the following primers were used to amplify the mouse IL-4 gene:
   forward primer: 5'-ccagctagttgtcatcctgctcttctttctcg-3' (SEQ ID NO: 3) and
   reverse primer: 5'-cagtgatgtggacttggactcattcatggtgc-3' (SEQ ID NO: 4), each of these primers being available from CLONTEC.
   As primers for amplifying a control mouse G3PDH gene, the following were used:
   forward primer: 5'-accacagtccatgccatcac-3' (SEQ ID NO: 5) and
   reverse primer: 5'-tccaccaccctgttgctgta-3' (SEQ ID NO: 6).

The results of measurement for the mRNA expression of IFN-γ and IL4 are shown in FIG. 4. In FIG. 4: lane 1 shows the result of RT-PCR performed using the positive control as a template; lane 2 shows the result of RT-PCR performed using as a template the total RNA derived from the negative control mice injected intraperitoneally with 200 µL of physiological saline; lane 3 shows the result of RT-PCR performed using as a template the total RNA derived from the mice immunized intraperitoneally with OVA-SucPG-liposomes; and lane 4 shows the result of RT-PCR performed using as a template the total RNA derived from the negative control mice immunized intraperitoneally with OVA-free SucPG-liposomes. As shown in FIG. 4, it became clear that the mRNA of IFN-γ which was an index of cellular immune reaction and the mRNA of IL-4 which was an index of humoral immune reaction were both expressed in the spleen lymphocytes from the mice immunized with the OVA-SucPG-liposomes.

In Example 3, the purified spleen lymphocytes were also cultured in an OVA-supplemented medium for 5 days and the amounts of IFN-γ and IL-4 that were released into the supernatant of the culture were measured as indices of cellular immunity and humoral immunity, respectively. To quantify the IFN-γ and IL-4 in the supernatant of the culture, the relevant quantitation kits (Endogen) were used.

The results of quantitation of the amounts of IFN-γ and IL-4 released into the supernatant of the culture of spleen lymphocytes are shown in FIG. 5. In FIG. 5: lane 1 shows the amount of IFN-γ or IL-4 as produced from the spleen lymphocytes derived from the negative control mice immunized intranasally with the OVA-free SucPG-liposomes, and lane 2 shows the amount of IFN-γ or IL-4 as produced from the spleen lymphocytes derived from the mice immunized intranasally with the OVA-SucPG-liposomes. As shown in FIG. 5, it became clear that, in the supernatant of the culture of the spleen lymphocytes from the mice immunized with the OVA-SucPG-liposomes, IFN-γ which was an index of cellular immune reaction and IL-4 which was an index of humoral immune reaction were both produced in statistically significantly high levels (p<0.0001).

From the results described in Examples 2 and 3, it has been shown that the vaccine in the SucPG-containing liposomes of the present invention, when it is used in transnasal immunization, can induce not only humoral immunity but also cellular immunity and this indicates that the SucPG-containing liposomes of the present invention are useful as an antigen carrier for transmucosal vaccines.

### Example 4: Study of Immune Response from Non-Transmucosal Administration of Vaccine in Succinylated Poly(glycidol) (SucPG) Liposomes

The purpose of this Example was to study the immune response from non-transmucosal administration of a vaccine in the SucPG-containing liposomes prepared in Example 1, as compared with a vaccine in the SucPG-free liposomes.

The vaccine in the SucPG-containing liposomes was prepared as described in Example 1. On the other hand, the vaccine in the SucPG-free liposomes was a vaccine in multilamellar vesicles (OVA-liposomes) (MLV) that were prepared by encapsulating OVA in a lipid composition consisting of DPPC and DOPE at a molar ratio of 1:1.

The thus prepared two types of vaccines, one in the OVA-SucPG-liposomes and the other in the OVA-liposomes, as well as a vaccine solely composed of OVA were administered intraperitoneally to BALB/c mice twice at a 7-day interval, each time to give 100 µg per mouse of OVA.

Seven days after the final administration of the immunogen, 0.1 ml of blood was taken from the orbital venous plexus and the serum collected from the blood was used to study the production of anti-OVA antibodies (IgM, IgG, and IgE) by the ELISA procedure. In addition, the immune serum obtained was used to make an analysis for anti-OVA-IgG subclasses by the ELISA procedure.

The results are shown in FIGs. 6 and 7. FIG. 6 shows the results for immune response from intraperitoneal immunization of the BALB/c mice with the vaccine in the OVA-SucPG-liposomes, the vaccine in the OVA-liposomes, and the vaccine solely composed of OVA; the black columns show the results of immunization with OVA only (OVA); the gray columns show the results of immunization with the OVA-liposomes (OVA-lipo); and the white columns show the results of immunization with the OVA-SucPG-liposomes (OVA-SucPG-lipo). FIG. 7 shows the results with the anti-OVA-IgG antibody subclasses and the immunogens associated with the respective columns are the same as explained above in connection with FIG. 6.

From the results shown in FIG. 6, it can be seen that when the BALB/c mice were immunized intraperitoneally with the variety of immunogens, antibodies of IgM and IgG classes were produced in the animal bodies, with the production of the IgG class antibody being generally greater than that of the IgM class antibody. For each class of antibody, the relationship between the type of vaccine carrier and the amount of antibody production was investigated; it was shown that, in comparison with the case of immunization with the vaccine solely composed of OVA, the case of immunization with the vaccine in the OVA-liposomes produced a greater amount of antibody but no significant difference was found as the result of a t test; on the other hand, a comparison between the case of immunization with the vaccine in the OVA-liposomes and the case of immunization with the vaccine in the OVA-SucPG-liposomes showed that the immunization with the vaccine in the OVA-SucPG-liposomes was capable of antibody production in a markedly high efficiency (p<0.0019).

In addition, the results shown in FIG. 7 indicate that immunization with the vaccine in the OVA-SucPG-liposomes could induce not only IgG1 which was an IgG subclass of Th2 (humoral immunity) type but also IgG2a and IgG3 which were IgG subclasses of Th1 (cellular immunity) type. On the other hand, when immunization was effected using the vaccine in the OVA-liposomes or the vaccine solely composed of OVA, IgG1 was practically all the antibody that cold be produced. Further, as for the amount of production of that IgG1 antibody, it was shown that immunization with the vaccine in the OVA-SucPG-liposomes was capable of antibody production in a markedly high efficiency (p<0.019) as compared with the case of immunization with the vaccines in other vaccine carriers (the vaccine in the OVA-liposomes and the vaccine solely composed of OVA).

Accordingly, the following general observations were obtained from the non-transmucosal administration of the vaccine in the SucPG-containing liposomes: it was capable of efficient antigen introduction into antigen-presenting cells, eventually inducing high antibody production; and it was potentially capable of inducing not only humoral immunity but also cellular immune response.

### Example 5: Induction of Cellular Immune Response from Non-Transmucosal Administration of Vaccine in SucPG-Containing Liposomes

Since it was shown in Example 4 that immunization with an antigen using the SucPG-containing liposomes had the potential to induce cellular immune response, Example 5 was conducted to study an ability to exert the cellular immune response for the case of using the SucPG-containing liposomes.

The OVA-SucPG-liposomes prepared in Example 1 were administered intraperitoneally to BALB/c mice twice at a 7-day interval, each time to give 100 µg per mouse of OVA. Seven days after the final administration, the mice were sacrificed and the spleen was collected and subjected to density-gradient centrifugation to purify the spleen lymphocytes. Using the thus purified spleen lymphocytes, IFN-γ and IL-4 were measured for mRNA expression by the RT-PCR technique and the amounts of IFN-γ and IL-4 released into the culture supernatant were measured by the ELISA procedure as described in Example 3.

The results of measurement for the mRNA expression of IFN-γ and IL4 are shown in FIG. 8. In FIG. 8: lane 1 shows the result of RT-PCR performed using as a template the total RNA derived from the mice immunized intraperitoneally with the OVA-SucPG-liposomes; lane 2 shows the result of RT-PCR performed using a positive control as a template; lane 3 shows the result of RT-PCR performed using as a template the total RNA derived from the negative control mice immunized intraperitoneally with OVA-free SucPG-liposomes; and lane 4 shows the result of RT-PCR performed using as a template the total RNA derived from the negative control mice injected intraperitoneally with 200 µL of physiological saline. As shown in FIG. 8, it became clear that the mRNA of IFN-γ which was an index of cellular immune reaction and the mRNA of IL-4 which was an index of humoral immune reaction were both expressed in the spleen lymphocytes from the mice immunized with the OVA-SucPG-liposomes.

In addition, the results of quantitation of the amounts of IFN-γ and IL-4 released into the supernatant of the culture of spleen lymphocytes are shown in FIG. 9. In FIG. 9: lane 1 shows the amount of IFN-γ or IL-4 as produced from the spleen lymphocytes derived from the negative control mice immunized intraperitoneally with the OVA-free SucPG-liposomes, and lane 2 shows the amount of IFN-γ or IL-4 as produced from the spleen lymphocytes derived from the mice immunized intraperitoneally with the OVA-SucPG-liposomes. As shown in FIG. 9, it became clear that, in the supernatant of the culture of the spleen lymphocytes from the mice immunized with the OVA-SucPG-liposomes, IFN-γ which was an index of cellular immune reaction and IL-4 which was an index of humoral immune reaction were both produced in statistically significantly high levels (p<0.0001).

From the results described in Examples 4 and 5, it has been shown that the vaccine in the SucPG-containing liposomes of the present invention, even when it is used in non-transnasal immunization, can induce not only humoral immunity but also cellular immunity as in the case of transnasal immunization and this indicates that the SucPG-containing liposomes of the present invention are also useful as an antigen carrier for non-transmucosal vaccines.

### Example 6: Ophthalmic (Transmucosal) Immunization of Chickens with Vaccine in Salmonella Antigen-SucPG-Liposomes

The purpose of this Example was to study the immune response from ophthalmic (transmucosal) administration to chickens of a vaccine in SucPG-containing liposomes containing *Salmonella enteritidis* antigen as an immunogen.

The *Salmonella enteritidis* antigen, or the immunogen to be used in this Example, was prepared in the following manner. First, *Salmonella enteritidis* (strain 1227) was inoculated in a heart infusion medium (Nissui Pharmaceutical Co., Ltd.) and following cultivation at 37°C for 14 hours, 7 x 10¹⁴ CFU of the bacterium *Salmonella enteritidis* was harvested. The harvested bacterium *Salmonella enteritidis* was inactivated by denaturation with an excess amount of formalin; following the removal of formalin, sonication was conducted to prepare an antigenic fluid. A vaccine in SucPG-containing liposomes containing the thus prepared *Salmonella enteritidis* antigen (vaccine in *Salmonella enteritidis* antigen-SucPG-liposomes) was prepared by a method that was basically the same as the procedure described in Example 1.

The thus prepared vaccine in *Salmonella enteritidis* antigen-SucPG-liposomes was administered once to 5 chickens (white leghorn) 3 weeks old after birth by dropping onto the eyes to give 100 µg per chick of *Salmonella enteritidis* antigen.

At days 14 and 35 after the administration of the immunogen (designated as "2 wks (5-wk old)" and "5 wks (8-wk old)", respectively), 2.0 mL of blood was collected from the wing vein (also called as basilic vein) and using the serum collected from the blood sample, the production of anti-*Salmonella enteritidis* antigen antibody (IgG or IgA) was studied by the ELISA procedure. As a control, there was used serum that had been obtained from chick individuals that were yet to be immunized with the *Salmonella enteritidis* antigen (designated as pre (3-wk old)).

The results are shown in FIG. 10. In FIG. 10, the black columns show the results for the antibody titer of the IgG antibody (FIG. 10A) and the white columns show the results for the antibody titer of the IgA antibody (FIG. 10B). The symbol "*" indicates the presence of a significant difference (p<0.0001) from the antibody titers of the antibodies in chick individuals before immunization (Day 0). The data show mean ± standard error.

From the results shown in FIG. 10, it can be seen that when chickens were immunized by ophthalmic administration of the vaccine in *Salmonella enteritidis* antigen-SucPG-liposomes, both IgG and IgA classes of antibody were produced markedly in the body in comparison with the case of "pre (3-wk old)" but from a long-term viewpoint (5 weeks (8-wk old) after the final administration of the immunogen), the IgG class of antibody tended to be produced in a greater amount than the IgA class of antibody.

From these results, it has been shown that immunizing chickens by ophthalmic (transmucosal) administration of the vaccine in *Salmonella enteritidis* antigen-SucPG-liposomes can induce high antibody production in the blood.

### Example 7: Transnasal (Transmucosal) Immunization of Mice with Vaccine in Trypanosoma Antigen-SucPG-Liposomes

The purpose of this Example was to study the immune response from transnasal (transmucosal) administration to mice of a vaccine in SucPG-containing liposomes containing *Trypanosoma brucei* antigen as an immunogen.

To obtain the *T. brucei* antigen, the immunogen to be used in this Example, protozoa *T. brucei* were collected. The method of collection was in accordance with a published method (Lanham, S. M., Nature, 218, 1273-1274 (1968)). Specifically, protozoa *T. brucei* (1 x 10⁵ parasites) were inoculated in the abdominal cavities of Wistar rats (Japan SLC, Inc.) and 4 days later, whole blood was collected from their hearts. The collected blood was treated with heparin (10 units/mL) for inhibition of protection against coagulation, and the buffy coat was collected by centrifuging (1300 g x 10 min). The protozoa were purified and harvested from the collected buffy coat by means of a DE52 cellulose (Whatman) column. The harvested protozoa *T. brucei* were ground by sonication to obtain the *T. brucei* antigen. A vaccine in SucPG-containing liposomes containing the thus prepared *T. brucei* antigen (vaccine in *T. brucei* antigen-SucPG-liposomes) was prepared by a method that was basically the same as the procedure described in Example 1.

The thus prepared vaccine in *T. brucei* antigen-SucPG-liposomes was administered transnasally to five BALB/c mice (Japan SLC, Inc.) 6 weeks old after birth to give 100 µg per mouse of *T. brucei* antigen, and 2 weeks later the same amount of *T. brucei* antigen was additionally boosted transnasally to effect immunization.

Fourteen days after the initial administration of the immunogen (Day 14) and seven days after the final administration of the immunogen (Day 21), 0.1 ml of blood was taken from the orbital venous plexus and the serum collected from the blood was used to study the production of anti-*T. brucei* antigen antibodies (IgG and IgM) by the ELISA procedure. As a control, there was used serum that had been obtained from mouse individuals that were yet to be immunized with the *T. brucei* antigen (Day 0).

The results are shown in FIG. 11. In FIG. 11, the black columns show the results for the antibody titer of the IgM antibody and the white columns show the results for the antibody titer of the IgG antibody. The symbol "#" indicates the presence of a significant difference at p<0.0012 from the antibody titers in mouse individuals before immunization (Day 0), the symbol "$" indicates the presence of a significant difference at p<0.0006, and the symbol "*" indicates the presence of a significant difference at p<0.0001. The data show mean ± standard error.

From the results shown in FIG. 11, it can be seen that when mice were immunized by transnasal administration of the vaccine in *T. brucei* antigen-SucPG-liposomes, both IgM and IgG classes of antibody were produced markedly in the body in comparison with the case of Day 0 and, in general, the IgG class of antibody tended to be produced in a greater amount than the IgM class of antibody.

From these results, it has been shown that immunizing mice by transnasal (transmucosal) administration of the vaccine in *T. brucei* antigen-SucPG-liposomes can induce high antibody production in the blood.

### Example 8: Transnasal (Transmucosal) Immunization of Milking Cows with Vaccine in Staphylococcus aureus Antigen-SucPG-Liposomes

The purpose of this Example was to study the immune response from transnasal (transmucosal) administration to cows of a vaccine in SucPG-containing liposomes containing *Staphylococcus aureus* antigen as an immunogen.

To prepare the *S. aureus* antigen, the immunogen to be used in this Example, *S*. *aureus* (strain Cowan I) was first inoculated in an LB medium (Nissui Pharmaceutical Co., Ltd.) and following cultivation at 37°C for 14 hours, the bacterium *S. aureus* was harvested. The harvested bacterium *S*. *aureus* was inactivated by denaturation with an excess amount of formalin; following the removal of formalin, sonication was conducted to prepare an antigenic fluid. A vaccine in SucPG-containing liposomes containing the thus prepared *S. aureus* antigen (vaccine in *S. aureus* antigen-SucPG-liposomes) was prepared by a method that was basically the same as the procedure described in Example 1.

The thus prepared vaccine in *S. aureus* antigen-SucPG-liposomes was administered transnasally to three Holstein milking cows to give 5 mg per cow of *S. aureus* antigen, and 14 days later the same amount of *S. aureus* antigen was additionally boosted transnasally to effect immunization.

Fourteen days after the initial administration of the immunogen (Day 14) and 7 and 14 days after the final administration of the immunogen (Day 21 and Day 28), 5 ml of blood was taken from the cervical vein and the serum collected from the blood was used to study the production of anti-*S. aureus* antigen antibodies (IgA and IgG) by the ELISA procedure. As a control, there was used serum that had been obtained from milking cow individuals that were yet to be immunized with the *S*. *aureus* antigen (Day 0).

The results are shown in FIG. 12. In FIG. 12, the black columns show the results for the antibody titer of the IgA antibody and the white columns show the results for the antibody titer of the IgG antibody. The symbol "#" indicates the presence of a significant difference at p<0.018 from the antibody titers in milking cow individuals before immunization (Day 0), the symbol "&" indicates the presence of a significant difference at p<0.039, the symbol "*" indicates the presence of a significant difference at p<0.0076, the symbol "@" indicates the presence of a significant difference at p<0.0001, and the symbol "t" indicates the presence of a significant difference at p<0.017. The data show mean ± standard error.

From the results shown in FIG. 12, it can be seen that when milking cows were immunized by transnasal administration of the vaccine in *S. aureus* antigen-SucPG-liposomes, almost comparable levels of IgG and IgA classes of antibody were produced markedly in the blood in comparison with the case of Day 0.

Fourteen days after the initial administration of the immunogen (Day 14) and 7 and 14 days after the final administration of the immunogen (Day 21 and Day 28), milk was collected from the cows and studied for the production of anti-*S*, *aureus* antigen antibodies (IgG and IgA) by the ELISA procedure. As a control, there was used milk that had been obtained from milking cow individuals that were yet to be immunized with the *S. aureus* antigen (Day 0).

The results are shown in FIG. 13. In FIG. 13, the black columns show the results for the antibody titer of the IgA antibody and the white columns show the results for the antibody titer of the IgG antibody. The symbol "#" indicates the presence of a significant difference at p<0.0046 from the antibody titers in milking cow individuals before immunization (Day 0), the symbol "&" indicates the presence of a significant difference at p=0.054, the symbol "*" indicates the presence of a significant difference at p<0.011, the symbol "@" indicates the presence of a significant difference at p<0.02, and the symbol "t" indicates the presence of a significant difference at p<0.025. The data show mean ± standard error.

From the results shown in FIG. 13, it can be seen that when milking cows were immunized by transnasal administration of the vaccine in *S. aureus* antigen-SucPG-liposomes, both IgG and IgA classes of antibody were produced markedly in the milk in comparison with the case of Day 0 and, in general, the IgA class of antibody tended to be produced in a greater amount than the IgG class of antibody.

From these results, it has been shown that immunizing milking cows by transnasal (transmucosal) administration of the vaccine in *S. aureus* antigen-SucPG-liposomes can induce markedly high antibody production in both blood and milk.

### Example 9: Oral Immunization of Carp with Vaccine in Aeromonas salmonicida Antigen-SucPG-Liposomes

The purpose of this Example was to study the immune response from oral (transmucosal) administration to carp of a vaccine in SucPG-containing liposomes containing *Aeromonas salmonicida* antigen as an immunogen.

The *A. salmonicida* antigen, or the immunogen to be used in this Example, was prepared in the following manner. First, *A. salmonicida* (strain T1031) was inoculated in a heart infusion medium and following cultivation at 20°C for 24 hours, the bacterium *A. salmonicida* was harvested and the harvested bacterium *A. salmonicida* was inactivated by denaturation with an excess amount of formalin; following the removal of formalin, sonication was conducted to prepare an antigenic fluid. A part of the *A. salmonicida* antigen was immediately utilized as an immunogen whereas another part was utilized to prepare a vaccine in SucPG-containing liposomes containing the *A. salmonicida* antigen. The vaccine in SucPG-containing liposomes containing the inactivated *A. salmonicida* antigen (vaccine in *A. salmonicida* antigen-SucPG-liposomes) was prepared by a method that was basically the same as the procedure described in Example 1.

The thus prepared vaccine in *A. salmonicida* antigen-SucPG-liposomes was orally administered three times at 2-wk intervals to six carp (distributed from Aquatic Life Conservation Research Center, Research Institute of Environment, Agriculture and Fisheries, Osaka Prefectural Government) for immunization to give 200 µg per carp of the *A. salmonicida* antigen. As a control, only the *A. salmonicida* antigen was orally administered three times at 2-wk intervals to four carp for immunization to give 200 µg per carp of the *A. salmonicida* antigen.

At the initial administration of the immunogen (Day 0), at its second administration (Day 14), at its third administration (Day 28) and 14 days after its final administration (Day 42), 1 ml of blood was taken from the caudal peduncle of each carp and the serum collected from the blood sample was used to study the production of anti-*A. salmonicida* antigen antibody by the ELISA procedure. As a control, there was used serum that had been obtained from carp individuals immunized with the *A. salmonicida* antigen only.

The results are shown in FIG. 14. In FIG. 14, the black circles (●) indicate the results for the antibody titer of the antibody in the carp orally immunized with the vaccine in *A. salmonicida* antigen-SucPG-liposomes whereas the black triangles (A) indicate the results for the antibody titer of the antibody in the carp orally immunized with the *A. salmonicida* antigen alone. The symbol "*" indicates the presence of a significant difference (p<0.01) from the antibody titer of the antibody in the carp orally immunized with the *A. salmonicida* antigen alone. The data show mean ± standard error.

From the results shown in FIG. 14, it can be seen that when carp were immunized by oral administration of the vaccine in *A. salmonicida* antigen-SucPG-liposomes, the antibody titer of the antibody in the blood was markedly enhanced in comparison with the carp that were orally immunized with the *A. salmonicida* antigen only.

Based on these results, 14 days after the final administration of the vaccine in *A. salmonicida* antigen-SucPG-liposomes (Day 42), the intestinal fluid and bile were collected from the carp and studied for the production of anti-*A. salmonicida* antigen antibody by the ELISA procedure. As controls, there were used the intestinal fluid and bile that had been obtained from non-immunized carp individuals.

The results are shown in FIG. 15 for the antibody titer of the antibody in the intestinal fluid (FIG. 15A) and for the antibody titer of the antibody in the bile (FIG. 15B). In each panel, the black column refers to the result for the antibody titer of the antibody in the non-immunized carp individuals whereas the white column refers to the result for the antibody titer of the antibody in the carp individuals immunized with the vaccine in *A. salmonicida* antigen-SucPG-liposomes. The symbol "*" indicates the presence of a significant difference (p<0.01) from the antibody titer of the antibody in the non-immunized carp individuals. The data show mean ± standard error.

From the results shown in FIG. 15, it can be seen that when carp were immunized by oral administration of the vaccine in *A. salmonicida* antigen-SucPG-liposomes, the antibody titer of the antibody was markedly enhanced in each of the intestinal fluid and the bile.

Furthermore, 14 days after the final administration of the vaccine in *A. salmonicida* antigen-SucPG-liposomes, six carp were immersed in a suspension of the bacterium A. *salmonicida* (1 x 10⁶ cfu/mL) for 60 minutes so that they would be attacked by *A. salmonicida;* the subsequent transitional change of their survival rate was recorded. As controls, eight non-immunized carp individuals were similarly treated and the subsequent transitional change of their survival rate was recorded.

The results are shown in FIG. 16. In FIG. 16, the black circles (●) indicate the transitional change of the survival rate of the carp orally immunized with the vaccine in *A. salmonicida* antigen-SucPG-liposomes whereas the black triangles (A) indicate the transitional change of the survival rate of the non-immunized carp. As it turned out, the survival rate of the carp orally immunized with the vaccine in *A. salmonicida* antigen-SucPG-liposomes was higher than the survival rate of the non-immunized carp.

From these results, it has been shown that even with the fish carp, immunization by oral (transmucosal) administration of the vaccine in *A. salmonicida* antigen-SucPG-liposomes can induce high antibody production in the blood.

### Example 10: Transnasal (Transmucosal) Immunization of Mice with Vaccine in Mycoplasma gallisepticum Antigen-SucPG-Liposomes

The purpose of this Example was to study the immune response from transnasal (transmucosal) administration to mice of a vaccine in SucPG-containing liposomes containing *Mycoplasma gallisepticum* antigen as an immunogen.

The *Mycoplasma gallisepticum* antigen, the immunogen to be used in this Example, was prepared in the following manner. First, *Mycoplasma gallisepticum* (strain S6) was inoculated in Fray medium (Difco) supplemented with a fresh yeast extract and following cultivation at 37°C for 48 hours or longer, 3.58 x 10¹¹ CFU of the bacterium *Mycoplasma gallisepticum* was harvested. The harvested bacterium *Mycoplasma gallisepticum* was inactivated by denaturation with an excess amount of formalin; following the removal of formalin, sonication was conducted to prepare an antigenic fluid. A vaccine in SucPG-containing liposomes containing the thus prepared *Mycoplasma gallisepticum* antigen (vaccine in *Mycoplasma gallisepticum* antigen-SucPG-liposomes) was prepared by a method that was basically the same as the procedure described in Example 1.

The thus prepared vaccine in *Mycoplasma gallisepticum* antigen-SucPG-liposomes was administered transnasally to five BALB/c mice (Japan SLC, Inc.) 5 weeks old after birth to give 100 µg per head of *Mycoplasma gallisepticum* antigen, and 2 weeks later the same amount of *Mycoplasma gallisepticum* antigen was additionally boosted transnasally to effect immunization.

Seven days after the final administration of the immunogen (Day 21), 0.1 ml of blood was taken from the orbital venous plexus and the serum collected from the blood was used to study the production of anti-*M. gallisepticum* antigen antibodies (IgG and IgA) by the ELISA procedure. As a control, there was used serum that had been obtained from mouse individuals that were yet to be immunized with the *M. gallisepticum* antigen (Day 0).

The results are shown in FIG. 17. FIG. 17 shows the results of antibody class induction in serum as regards the immune response from transnasal administration of the vaccine in *M. gallisepticum* antigen-SucPG-liposomes to mice; the black columns show the results for the antibody titer of the IgG antibody and the white column shows the results for the antibody titer of the IgA antibody.

From the results shown in FIG. 17, it can be seen that when mice were immunized by transnasal administration of the *M. gallisepticum* antigen, both IgG and IgA classes of antibody were produced in the body and, in general, the IgG class of antibody tended to be produced in a greater amount than the IgA class of antibody. The symbol "#" indicates the presence of a significant difference at p<0.0063 from the antibody titers in the mouse individuals immediately before immunization with the *M. gallisepticum* antigen (Day 0) and the symbol "*" indicates the presence of a significant difference at p<0.0003. The data show mean ± standard error.

Accordingly, it has been shown that transmucosal administration of the vaccine of the *M. gallisepticum* antigen by means of the SucPG-containing liposomes can induce high antibody production in the blood and that it is potentially capable of inducing not only humoral immunity but also cellular immune response.

### Example 11: Transnasal (Transmucosal) Immunization of Mice with Vaccine in Newcastle Disease Virus Antigen-SucPG-Liposomes

The purpose of this Example was to study the immune response from transnasal (transmucosal) administration to mice of a vaccine in SucPG-containing liposomes containing Newcastle disease virus antigen as an immunogen.

The Newcastle disease virus antigen, the immunogen to be used in this Example, was prepared from a commercial live vaccine of Newcastle disease which was sonicated to prepare an antigenic fluid. A vaccine in SucPG-containing liposomes containing the thus prepared Newcastle disease virus antigen (vaccine in Newcastle disease virus antigen-SucPG-liposomes) was prepared by a method that was basically the same as the procedure described in Example 1.

The thus prepared vaccine in Newcastle disease virus antigen-SucPG-liposomes was administered transnasally to five BALB/c mice (Japan SLC, Inc.) 5 weeks old after birth to give 100 µg per head of Newcastle disease virus antigen, and 2 weeks later the same amount of Newcastle disease virus antigen was additionally boosted transnasally to effect immunization.

Seven days after the final administration of the immunogen (Day 21), 0.1 ml of blood was taken from the orbital venous plexus and the serum collected from the blood was used to study the production of anti-Newcastle disease virus antigen antibodies (IgG and IgA) by the ELISA procedure. As a control, there was used serum that had been obtained from mouse individuals that were yet to be immunized with the Newcastle disease virus antigen (Day 0).

The results are shown in FIG. 18. In FIG. 18, the black column shows the result for the antibody titer of the IgG antibody and the white columns show the results for the antibody titer of the IgA antibody. The symbol "#" indicates the presence of a significant difference at p<0.0027 from the antibody titers in the mouse individuals before immunization (Day 0) and the symbol "*" indicates the presence of a significant difference at p<0.00122. The data show mean ± standard error.

From the results shown in FIG. 18, it can be seen that when mice were immunized by transnasal administration of the vaccine in Newcastle disease virus antigen-SucPG-liposomes, both IgG and IgA classes of antibody were markedly produced in the body as compared with the case of Day 0 and, in general, the IgG class of antibody tended to increase in a greater degree than the IgA class of antibody.

From these results, it has been shown that immunizing the mice by transnasal (transmucosal) administration of the vaccine in Newcastle disease virus antigen-SucPG-liposomes can induce high antibody production in the blood.

### INDUSTRIAL APPLICABILITY

By using the above-described vaccine carriers that comprise liposomes containing succinylated poly(glycidol), efficient vaccines can be obtained that achieve marked increases in antibody titers as compared with the case of using vaccine carriers that comprises the conventional liposomes. In addition, the vaccines prepared by using the above-described vaccine carriers are capable of efficient induction of not only humoral immunity but also cellular immunity.

## Claims

1. A vaccine carrier comprising a liposome containing succinylated poly(glycidol).

2. The vaccine carrier according to claim 1 which contains 10 to 40 wt% of succinylated poly(glycidol).

3. The vaccine carrier according to claim 2 which contains 30 wt% of succinylated poly(glycidol).

4. The vaccine carrier according to any one of claims 1 to 3, wherein the lipid that composes the liposome comprises any one of dioleyl phosphatidylethanolamine (DOPE), distearoyl phosphatidylethanolamine (DSPE), dipalmitoyl phosphatidylserine (DPPS), dipalmitoyl phosphatidylcholine (DPPC), distearoyl phosphatidylcholine (DSPC), dimyristoyl phosphatidylcholine (DMPC), yolk lecithin (egg PC), or cholesterol, or combinations of any two or more thereof.

5. The vaccine carrier according to any one of claims 1 to 4, wherein the liposome comprises the combination of dioleyl phosphatidylethanolamine (DOPE) and distearoyl phosphatidylethanolamine (DSPE).

6. The vaccine carrier according to any one of claims 1 to 5, which enables the immunogen contained in the liposome to be internalized within an antigen-presenting cell.

7. The vaccine carrier according to any one of claims 1 to 6, which is for transmucosal administration of the immunogen contained in the liposome.

8. The vaccine carrier according to any one of claims 1 to 6, which is for non-transmucosal administration of the immunogen contained in the liposome.

9. A vaccine having an immunogen incorporated in a vaccine carrier comprising a liposome containing succinylated poly(glycidol), which immunogen is to be administered for immunization.

10. The vaccine according to claim 9, which is for inducing cellular immunity against the immunogen.

11. The vaccine according to claim 10, which is also for inducing humoral immunity against the immunogen.

12. The vaccine according to any one of claims 9 to 11, which contains 10 to 40 wt% of succinylated poly(glycidol) in the vaccine carrier.

13. The vaccine according to claim 12 which contains 30 wt% of succinylated poly(glycidol) in the vaccine carrier.

14. The vaccine according to any one of claims 9 to 13, wherein the lipid that composes the liposome of the vaccine carrier comprises any one of dioleyl phosphatidylethanolamine (DOPE), distearoyl phosphatidylethanolamine (DSPE), dipalmitoyl phosphatidylserine (DPPS), dipalmitoyl phosphatidylcholine (DPPC), distearoyl phosphatidylcholine (DSPC), dimyristoyl phosphatidylcholine (DMPC), yolk lecithin (egg PC), or cholesterol, or combinations of any two or more thereof.

15. The vaccine according to any one of claims 9 to 14, wherein the liposome of the vaccine carrier comprises the combination of dioleyl phosphatidylethanolamine (DOPE) and distearoyl phosphatidylethanolamine (DSPE).

16. The vaccine according to any one of claims 9 to 15, which enables the immunogen contained in the liposome to be internalized within an antigen-presenting cell.

17. The vaccine according to any one of claims 9 to 16, which is for transmucosal administration of the immunogen.

18. The vaccine according to any one of claims 9 to 16, which is for non-transmucosal administration of the immunogen.

19. The vaccine according to any one of claims 9 to 18, wherein the immunogen is selected from the group consisting of antigens derived from any of a bacterium, a virus, and a protozoan.

20. The vaccine according to claim 19, wherein the bacterium is selected from among *Salmonella, Staphylococcus aureus, Aeromonas,* and *Mycoplasma.*

21. The vaccine according to claim 19, wherein the virus is Newcastle disease virus.

22. The vaccine according to claim 19, wherein the protozoan is *Trypanosoma.*
